(19) Europäisches Patentamt
European Patent Office
Office européen des brevets

(11) Publication number: 0 371 733 A2

(12) # EUROPEAN PATENT APPLICATION

(21) Application number: 89312297.8

(51) Int. Cl.5: C07D 277/16, A61K 31/425

(22) Date of filing: 27.11.89

(30) Priority: 01.12.88 US 278637

(43) Date of publication of application: 06.06.90 Bulletin 90/23

(84) Designated Contracting States: AT BE CH DE ES FR GB GR IT LI LU NL SE

(71) Applicant: SMITHKLINE BEECHAM CORPORATION
P.O. Box 7929 1 Franklin Plaza
Philadelphia Pennsylvania 19101(US)

(72) Inventor: Kruse, Lawrence Ivan
9 Great Valley Parkway
Malvern Pennsylvania 19355(US)
Inventor: Ross, Stephen Torey
718 Old State Road
Berwyn Pennsylvania 19312(US)

(74) Representative: Waters, David Martin, Dr. et al
Smith Kline & French Laboratories Ltd.
Patent Department Mundells
Welwyn Garden City Hertfordshire AL7 1EY(GB)

(54) **Dopamine-beta-hydroxylase inhibitors.**

(57) Dopamine-β-hydroxylase inhibitors of structure:

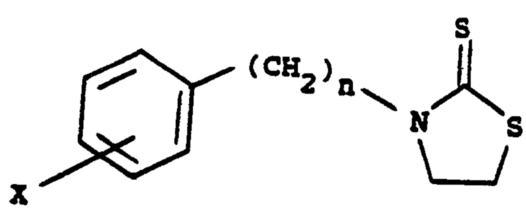

Pharmaceutical compositions containing them and their use in methods of lowering blood pressure in mammals.

EP 0 371 733 A2

Xerox Copy Centre

## DOPAMINE-β-HYDROXYLASE INHIBITORS

### FIELD OF THE INVENTION

This invention relates to novel compounds that inhibit dopamine-β-hydroxylase.

### BACKGROUND OF THE INVENTION

In the catecholamine biosynthetic pathway, tyrosine is converted in three steps to norepinephrine (NE). Intermediates are dihydroxyphenylalanine (DOPA) and dopamine (DA). Dopamine is hydroxylated to norepinephrine by dopamine-β-hydroxylase (DBH) in the presence of oxygen and ascorbic acid.

Inhibition of catecholamine activity decreases blood pressure. Weinshilboum, Mayo Clin. Proc. 55, 39 (1980), reviews compounds that inhibit catecholamine activity by acting upon adrenergic receptors. Alternatively, the catecholamine biosynthetic pathway can be suppressed at any of the three steps, resulting in reduced NE levels. In addition to producing an antihypertensive effect, inhibitors of NE synthesis are active as diuretics, natriuretics, cardiotonics, and vasodilators. Inhibition of DBH activity can have the added advantage of increasing DA levels, which as reported by Ehrreich et al., "New Antihypertensive Drugs," Spectrum Publishing, 1976, pp.409-432, has selective vasodilator activity at certain concentrations.

DBH inhibitors also have been shown to reduce or prevent formation of gastric ulcers in rats by Hidaka et al., "Catecholamine and Stress," edit. by Usdin et al., Permagin Press, Oxford, 1976, pp.159-165 and by Osumi et al., Japan J. Pharmacol. 23, 904 (1973).

A number of DBH inhibitors are known. These generally are divided into two classes, namely, metal chelating agents. which bind copper in the enzyme, and phenethylalamine analogues. Rosenberg et al., "Essays in Neurochemistry and Neuropharmacology," Vol. 4, ed. by Youdim et al., John Wiley & Sons, 1980, pp. 179-192, and Goldstein, Pharmacol. Ref. 18(1), 77 (1966), review DBH inhibitors.

Known DBH inhibitors include:

(a) 5-alkylpicolinic acids [See, Suda et al., Chem. Pharm. Bull. 17, 2377 (1969): Umezawa et al., Biochem. Pharmacol. 19, 35 (1969); Hidaka et al., Mol. Pharmacol. 9, 172 (1973); Miyano et al., Chem. Pharm. Bull. 26, 2328 (1978); Miyano et al., Heterocycles 14, 755 (1980): Claxton et al., Eur. J. Pharmacol. 37, 179 (1976)];

(b) BRL 8242 [see Claxton et al., Eur. J. Pharmacol. 37, 179 (1976)];

(c) 1-alkylimidazole-2-thiols [See, Hanlon et al., Life Sci. 12, 417 (1973); Fuller et al., Adv. Enzyme Regul. 15, 267 (1976)];

(d) substituted thioureas [See, Johnson et al., J. Pharmacol. Exp. Ther. 168, 229 (1969)]; and

(e) benzyloxymaine and benzylhydrazine [See, Creveling et al., Biochim. Biophys. Acta 64, 125 (1962); Creveling et al., Biochim. Biophys. Acta 8, 215 (1962); Van Der Schoot et al., J. Pharmacol. Exp. Ther. 141, 74 (1963); Bloom, Ann. N.Y. Acad. Sci. 107, 878 (1963)];

(f) fusaric acid derivatives and analogues as reported by Runti et al. in Il Farmaco Ed. Sci. 36, 260 (1980). These derivatives include phenylpicolinic acid, which has twice the inhibitory activity of fusaric acid, and 5-(4-chlorobutyl) picolinic acid, and others such as substituted amides of fusaric acid and acids and amides of 5-butyroylpicolinic acid, 5-aminopicolinic acid and 5-hydrazinopicolinic acid, and derivatives thereof:

(g) 5-(3,4-dibromobutyl)picolinic acid and 5-(dimethyldithiocarbamoylmethyl)picolinic acid (Hidaka et al., Molecular Pharmacology 9, 172-177, 1972);

(h) Bupicomide, 5-(n-butyl)picolinamine, Ehrreich et al., "New Antihypertensive Drugs", Spectrum Publications, 1976, pg. 409-432, reported as a DBH inhibitor that has antihypertensive activity;

(i) a series of 1-phenyl and 1-phenylalkylimidazole compounds having a mercapto or alkylthio group in the 2-position reported in European Patent Application No. 125,033 (published November 14, 1984);

(j) Methylpyridine derivatives isolated from the fermentation broth of a strain of Streptoverticillium (United States Patent No. 4,487,761); and

(k) 1-Benzyl-2-aminomethylimidazole derivatives (United States Patent No. 4,532,331).

However, non-specific, often toxic effects to known DBH inhibitors have obviated clinical use of these compounds. Fusaric acid, for example, is hepatotoxic. See, for example, Teresawa et al., Japan. Cir. J. 35,

339 (1971) and references cited therein.

Certain substituted thiazolidinethiones are known in the art :

(i) US patent 3,474,045 discloses 3-phenyl- and 3-benzylthiazolidinethiones as vulcanisation accelerators;

(ii) US patent 3,370,051 discloses further 3-phenylthiazidinethiones in which the phenyl group is substituted by a 2- or 4-methyl group or a 2- or 4-methoxy group; 3-benzylthiazolidinethiones in which the benzyl group is substituted by a 4-chloro, 4-methyl or 4-methoxy group; and 3-phenethylthiazolidinethiones; again as vulcanisation accelerators;

(iii) Further, 3-benzyl- and 3-phenethylthiazolidinethione are also disclosed in Arch. Exp. Veterinaermed, Weiffen et. al., 1967, 21, 1049 and indicated to have poor activity in an in vitro screen for tuberculostatic activity.

None of the foregoing references disclose any DBH inhibitory activity or suggest they would be of use in medicine. It has now been found that certain 3-substituted-2-thiazolidinethiones are also inhibitors of DBH activity, and are useful in the treatment of elevated blood pressure in mammals.

## SUMMARY OF THE INVENTION

The present invention relates to 3-aryl and aralkyl-2-thiazolidinethiones which have been found to be potent and prolonged inhibitors of DBH.

Presently preferred compounds of the invention include
3-(3-fluorophenylmethyl)-2-thiazolidinethione, and
3-(3,5-difluorophenylmethyl)-2-thiazolidinethione.

In addition the invention relates to pharmaceutical compositions comprising the compounds of the invention and a method of inhibiting DBH activity in mammals, including humans, which comprises administering internally to a subject an effective amount of a compound of the invention.

## DETAILED DESCRIPTION OF THE INVENTION

The present invention relates to a compound of structure (I) :

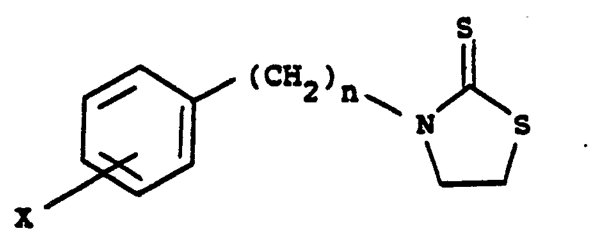

(I)

in which

X is hydrogen, halogen, $C_{1-4}$alkyl, $C_{1-4}$alkoxy, OH, CN, $NO_2$, $SO_2NH_2$, CHO, $CONH_2$, $CO_2H$, $CO_2C_{1-4}$alkyl, $CH_2OH$, $CF_3$, $SO_2C_{1-4}$alkyl or $SO_2C_mF_{2m+1}$ where m is 1 to 4, or any synthetically accessible combination thereof up to 4 substituents; and

n is 0 to 5;

or a hydrate thereof, provided that

(i) when n is O, X is other than hydrogen, 2-or 4-$C_{1-4}$alkyl, 2 or 4 $C_{1-4}$alkoxy or 4-halogen;

(ii) when n is 1, X is other than hydrogen, 2-or 4-halogen, 2,4-halogen, 3,4-halogen, 4-$C_{1-4}$alkyl or 4-$C_{1-4}$alkoxy; and

(iii) when n is 2, X is other than hydrogen.

Suitably X is hydrogen, halogen, $C_{1-4}$alkyl, $C_{1-4}$alkoxy, OH, CN, $NO_2$, $SO_2NH_2$, CHO, $CONH_2$, $CO_2H$, $CO_2C_{1-4}$alkyl, $CH_2OH$, $CF_3$, $SO_2C_{1-4}$alkyl or $SO_2C_mF_{2m+1}$, where m is 1 to 4, or any synthetically accessible combinations thereof up to 4 substituents. Preferably X is one or two substituents selected from the foregoing; most preferably X is one or two halogen substituents, in particular, fluorine.

Suitably, n is 0 to 5; preferably n is 1 or 3; most preferably n is 1.

As used herein, "synthetically accessible combination thereof" means any combination of the substituents that is available by chemical synthesis and is stable.

$C_{1-4}$alkyl either alone or as part of another group e.g. $C_{1-4}$alkoxy means a straight or branched chain alkyl having from 1 to 4 carbons.

The compounds of structure (I) can be prepared by processes analogous to those known in the art, in particular compounds of structure (I) can be prepared by reacting a compound of structure (II) :

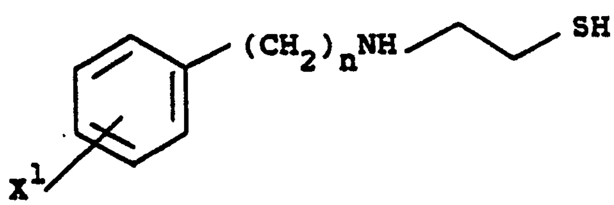

(II)

in which $X^1$ is as X described for structure (I) but not hydroxy with an alkali metal xanthate of structure (III)

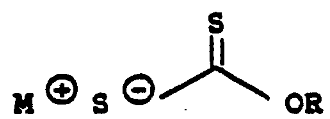

(III)

in which M is an alkali metal anion and R is $C_{1-4}$alkyl. Suitable alkali metal anions include, for example, sodium and potassium. Suitable groups R in structure (III) include methyl and ethyl. Preferably, the compound of structure (III) is potassium ethyl xanthate.

The reaction between a compound of structure (II) and a compound of structure (III) is carried out at elevated temperature in a suitable solvent. In particular the reaction is carried out at reflux temperature in a $C_{1-4}$alkanol, preferably ethanol.

Compounds of structure (II) can themselves be prepared reaction of compounds of structure (IV) :

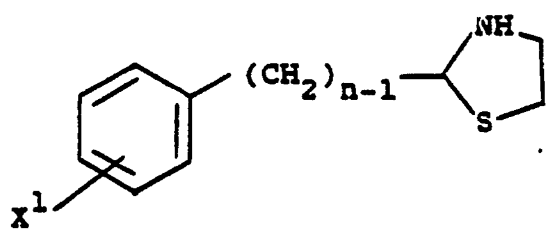

(IV)

in which $X^1$ and n are as described for structure (II), with a suitable reducing agent. Preferably, the reaction is carried out in an inert solvent, such as tetrahydrofuran, diethyl ether or dioxan and utilises lithium aluminum hydride. Other suitable reducing agents will be apparent to those skilled in the art.

Compounds of structure (IV) can be prepared from reaction between 2-mercaptoethylamine and an aldehyde of structure (V) :

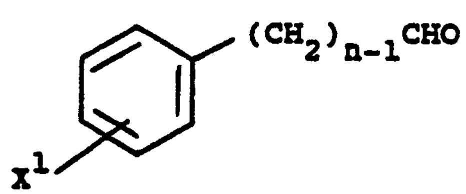

(V)

in which $X^1$ and n are as described for structure (II).

Suitably, the reaction is carried out at ambient temperature in an aqueous $C_{1-4}$alkanol in particular, ethanol, as solvent.

It will be appreciated that compounds of structure (I) in which X is hydroxy can be prepared from

compounds of structure (I) in which X is $C_{1-4}$alkoxy e.g. methoxy by using known hydrolysis methods, for example by treatment with boron tribomide or hydrogen bromide in an appropriate solvent.

The compounds of structure (IA):

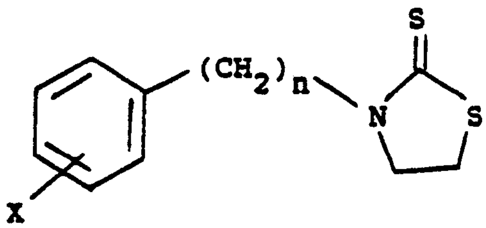

(IA)

in which

X is hydrogen, halogen, $C_{1-4}$alkyl, $C_{1-4}$alkoxy, OH, CN, $NO_2$, $SO_2NH_2$, CHO, $CONH_2$, $CO_2H$, $CO_2C_{1-4}$alkyl, $CH_2OH$, $CF_3$, $SO_2C_{1-4}$alkyl or $SO_2C_mF_{2m+1}$ where m is 1 to 4, or any synthetically accessible combination thereof up to 4 substituents; and

n is 0 to 5;

and hydrates thereof have been found to be potent and long lasting inhibitors of DBH activity, and as such they are useful as diuretic, natriuretic, cardiotonic, antihypertensive, and vasodilator agents, as well as anti-ulcerogenic and anti-Parkinsonian agents.

In a further aspect the present invention therefore provides a method of inhibiting DBH activity in mammals which comprises administering to a subject in need thereof an effective amount of a compound of structure (IA).

Listed in Table I are compounds of the invention that were tested for in vitro DBH inhibition by a standard procedure for assaying conversion of tyramine to octopamine in the presence of DBH. J.J. Pisano, et al., Biochim. Biophys. Acta, 43, 566-568 (1960). Octopamine was assayed following sodium periodate oxidation to p-hydroxybenzaldehyde by measuring spectrophotometric absorbence at 330 nm. In Table I, inhibition is given in molar concentration of compound at which DBH activity was halved ($IC_{50}$). Fusaric acid, by this test has an $IC_{50}$ of 8 x $10^{-7}$M.

Table I

| Compound | DBH $IC_{50}$ (μM) |
|---|---|
| 3-Phenylmethyl-2-thiazolidinethione | 72 ± 13 |
| 3-(3-Fluorophenylmethyl)-2-thiazolidinethione | 30 ± 3 |
| 3-(3,5-Difluorophenylmethyl)-2-thiazolidinethione | 13 ± 1 |

Further, spontaneously hypertensive rats were treated with 3-(3-fluorophenylmethyl)-2-thiazolidinethione at a dose of 50 mg/kg intraperitoneally, and mean arterial blood pressure was monitored for 250 minutes using indwelling cannulae in the tail arteries. When compared to vehicle-treated controls, the animals treated with this compound exhibited significant blood pressure reductions within 30 minutes following treatment and exhibited their lowest blood pressures when monitoring was discontinued. The maximal blood pressure reduction was approximately 20 mmHg.

When used in the method of the present invention the compounds are incorporated into standard pharmaceutical compositions. In a further aspect the present invention provides pharmaceutical compositions comprising a compound of structure (IA) or a hydrate thereof in association with a pharmaceutically acceptable carrier.

The compounds of structure (IA) can be incorporated into convenient pharmaceutical dosage forms such as capsules, tablets, or injectable preparations. Solid or liquid pharmaceutical carriers can be employed. Solid carriers include, starch, lactose, calcium sulfate dihydrate, terra alba, sucrose, talc, gelatin, agar, pectin, acacia, magnesium stearate, and stearic acid. Liquid carriers include syrup, peanut oil, olive oil, saline, and water. Similarly, the carrier or diluent may include any prolonged release material, such as glyceryl monostearate or glyceryl distearate, along or with a wax. The amount of solid carrier varies widely

but, preferably, will be from about 25 mg to about 1 g per dosage unit. When a liquid carrier is used, the preparation will be in the form of a syrup, elixir, emulsion, soft gelatin capsule, sterile injectable liquid, or an aqueous or nonaqueous liquid suspension.

The pharmaceutical preparations are made following conventional techniques of a pharmaceutical chemist involving mixing, granulating and compressing, when necessary, for tablet forms, or mixing, filling, and dissolving the ingredients, as appropriate, to give the desired oral or parenteral products.

Doses of the present compounds of structure (IA) in a pharmaceutical dosage unit as described above will be an efficacious, non-toxic quantity selected from the range of 0.1-100 mg/kg of active compound, preferably 0.1-50 mg/kg. The selected dose is administered to a human patient in need of DBH inhibition from 1-6 times daily, orally, rectally, by injection, or continuously by infusion. Oral dosage units for human administration preferably contain from 1 to 500 mg of active compound. Parenteral administration, which uses lower dosages is preferred. Oral administration, as higher dosages, however, also can be used when safe and convenient for the patient.

The following examples serve to illustrate the invention.

## Example 1

### 3-Phenylmethyl-2-thiazolidinethione

10.0 g (0.0943 mol) of benzaldehyde, 11.8 g (0.1037 mol) of 2-mercaptoethylamine hydrochloride and 4.15 g (0.1037 mol) of sodium hydroxide were added to a mixture of ethanol (100 ml) and water (50 ml) and a complete solution obtained by stirring and warming. A crystalline solid formed. The reaction mixture was stirred for 30 minutes at ambient temperature and then was filtered to give a crystalline solid, 2-phenyltetrahydrothiazole, 12.9 g (86%) after drying. This material (0.079 mol) was suspended in tetrahydrofuran (THF) (50 ml) and 76 ml (0.076 mol) of 1.0 M lithium aluminum hydride (LAH) in THF was added dropwise with stirring. The tetrahydrothiazole dissolved during the addition period and a 10° C exotherm occurred which was controlled by application of an ice bath. Following the LAH addition the reaction mixture was heated briefly to reflux and then was cooled to ambient temperature and water was added dropwise until an excess was present and remaining reagent had been destroyed. The aqueous mixture was filtered and the filtrate was neutralized to pH 7 with acetic acid and then extracted three times with ethyl acetate. The combined ethyl acetate extracts were concentrated to an oily residue which was triturated with ether. The triturate was concentrated to yield 5.4 g (41%) of N-benzyl-2-mercaptoethylamine as an oil. This material (0.0323 mol) and 5.18 g (0.0323 mol) of potassium ethyl xanthate were dissolved in 50 ml of 95% ethanol with stirring and the mixture was refluxed for 16 hours and then was cooled, diluted with an equal portion of water and then was neutralised to pH 7 with acetic acid. The aqueous mixture was then extracted three times with ethyl acetate and the combined ethyl acetate extracts concentrated to give a crystalline solid. This was recrystallised from ethyl acetate to give 3-phenylmethyl-2-thiazolidinethione, 2.6 g (38%), m.p. 133.5-134.5° C. Elemental analysis (C,H,N,S) and infrared and ′HNMR spectra were consistent with structure. (This compound is described by Giumanini and Plessi, J. Prakt. Chem, 1977, 319, 837-9, and can also be prepared by the method described therein).

## Example 2

### 3-(3-Fluorophenylmethyl)-2-thiazolidinethione

Using the procedure of Example 1, substituting 3-fluorobenzaldehyde for benzaldehyde gave the title compound in 13% overall yield, m.p. 93.5-95° C. Elemental analyses (C,H,N,S) and infrared and ′HNMR spectra were consistent with structure.

Example 3

3-(3,5-Difluorophenylmethyl)-2-thiazolidinethione

Using the procedure of Example 1, substituting 3,5-difluorobenzaldehyde for benzaldehyde gave the title compound in 13% overall yield, m.p. 107-109°C. Elemental analyses (C,H,N,S) and infrared and ′HNMR spectra were consistent with structure.

Example 4

3-3-(Phenylpropyl)-2-thiazolidinethione

Using the procedure of Example 1, substituting 3-phenylpropanal yields the title compound.

Example 5

3-(3-Methoxyphenylethyl)-2-thiazolidinethione

Using the procedure of Example 1, substituting 3-methoxyphenylethanol yields the title compound.

Example 6

3-(3-Trifluorophenylmethyl)-2-thiazolidinethione

Using the procedure of Example 1, substituting 3-trifluoromethylbenzaldehyde yields the title compound.

Example 7

3-(3,5-Difluoro-4-methoxyphenylmethyl)-2-thiazolidinethione

Using the procedure of Example 1, substituting 3,5-difluoro-4-methoxybenzaldehyde yields the title compound.

Example 8

3-(3,5-Difluoro-4-hydroxyphenylmethyl)-2-thiazolidinethione

The reaction of 3-(3,5-difluoro-4-methoxyphenylmethyl)-2-thiazolidinethione with $BBr_3$ in $CH_2Cl_2$ followed by aqueous workup yields the title compound.

Example 9

3-(3-chlorophenylmethyl)-2-thiazolidinethione

The reaction of 3-chlorobenzaldehyde using the procedure of Example 1 yields the title compound.

Example 10

3-4-(phenylbutyl)-2-thiazolidinethione

The reaction of 4-phenylbutanal according to the procedure of Example 1 yields the title compound.

Example 11

An oral dosage form for administering the present invention compounds is produced by screening, mixing, and filling into hard gelatin capsules the ingredients in the proportions shown in Table II, below:

Table II

| Compound | Amounts |
|---|---|
| 3-(3-Fluorophenylmethyl)-2-thiazolidinethione | 50 mg |
| magnesium stearate | 5 mg |
| lactose | 75 mg |

Example 12

The sucrose, calcium sulfate dihydrate, and compound shown in Table III below, are mixed and granulated in the proportions shown with a 10% gelatin solution. The wet granules are screened, dried, mixed with the starch, talc and stearic acid, screened and compressed into a tablet.

Table III

| Compound | Amounts |
|---|---|
| 3-(3-Fluorophenylmethyl)-2-thiazolidinethione | 150 mg |
| sucrose | 20 mg |
| starch | 10 mg |
| talc | 5 mg |
| stearic acid | 3 mg |

## Example 13

3-(3-Fluorophenylmethyl)-2-thiazolidinethione is dispersed in 25 ml of normal saline to prepare an injectable preparation.

## Claims

1. A compound of the formula (I):

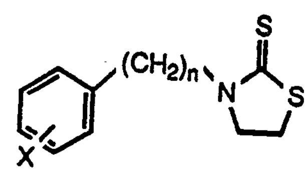

(I)

in which
X is hydrogen, halogen, $C_{1-4}$alkyl, $C_{1-4}$alkoxy, OH, CN, $NO_2$, $SO_2NH_2$, CHO, $CONH_2$, $CO_2C_{1-4}$alkyl, $CO_2H$, $CH_2OH$, $CF_3$, $SO_2C_{1-4}$alkyl, or $SO_2C_mF_{2m+1}$ where m is 1 to 4, or any synthetically accessible combination thereof up to 4 substituents; and n is 0 to 5;
or a hydrate thereof, provided that
(i) when n is 0, X is other than hydrogen, 2- or 4-$C_{1-4}$alkyl, 2- or 4-$C_{1-4}$alkoxy or 4-halogen;
(ii) when n is 1, X is other than hydrogen, 2- or 4-halogen, 2,4-halogen, 3,4-halogen, 4-$C_{1-4}$alkyl or 4-$C_{1-4}$alkoxy; and
(iii) when n is 2, X is other than hydrogen.

2. A compound according to claim 1 in which n is 1.
3. A compound according to claim 1 or 2 in which X is one or two halogen substituents.
4. A compound according to claim 1 which is 3-(3-fluorophenylmethyl)-2-thiazolidinethione or 3-(3,5-difluorophenylmethyl)-2-thiazolidinethione; or a hydrate thereof.
5. A compound according to any one of claims 1-4 for use as a medicament.
6. A pharmaceutical composition comprising a compound of formula (IA):

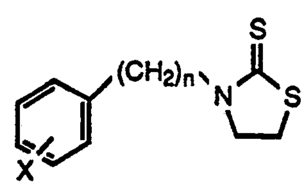

(IA)

in which
X is hydrogen, halogen, $C_{1-4}$alkyl, $C_{1-4}$alkoxy, OH, CN, $NO_2$, $SO_2NH_2$, CHO, $CONH_2$, $CO_2C_{1-4}$alkyl, $CO_2H$, $CH_2OH$, $CF_3$, $SO_2C_{1-4}$alkyl, or $SO_2C_mF_{2m+1}$ where m is 1 to 4, or any synthetically accessible

combination thereof up to 4 substituents; and
n is 0 to 5;
or a hydrate thereof, in association with a pharmaceutically acceptable carrier.

7. The pharmaceutical composition of claim 6 in which the compound is 3-(3-fluorophenylmethyl)-2-thiazolidinethione or 3-(3,5-difluorophenylmethyl)-2-thiazolidinethione.

8. A process for preparing a compound of formula (I) or a hydrate thereof as defined in claim 1, which process comprises reacting a compound of formula (II):

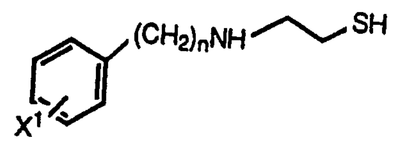

(II)

in which $X^1$ is as described for formula (I) but not hydroxy with a xanthate of formula (III):

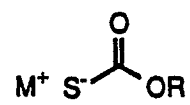

(III)

in which $M^+$ is an alkali metal ion and R is $C_{1-4}$alkyl and when compounds wherein X is OH are desired, deprotecting the compounds wherein $X^1$ is $C_{1-4}$alkoxy.

9. The use of a compound of the formula (IA) or a hydrate thereof as defined in claim 6 in the manufacture of a medicament for inhibiting dopamine-$\beta$-hydroxylase.

Claims for the following Contracting States: ES and GR

1. A process for the preparation of a compound of formula (I):

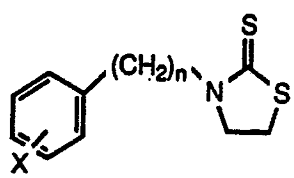

(I)

in which
X is hydrogen, halogen, $C_{1-4}$alkyl, $C_{1-4}$alkoxy, OH, CN, $NO_2$, $SO_2NH_2$, CHO, $CONH_2$, $CO_2C_{1-4}$alkyl, $CO_2H$, $CH_2OH$, $CF_3$, $SO_2C_{1-4}$alkyl, or $SO_2C_mF_{2m+1}$ where m is 1 to 4, or any synthetically accessible combination thereof up to 4 substituents; and
n is 0 to 5;
or a hydrate thereof, provided that
(i) when n is 0, X is other than hydrogen, 2- or 4-$C_{1-4}$alkyl, 2- or 4-$C_{1-4}$alkoxy or 4-halogen;
(ii) when n is 1, X is other than hydrogen, 2- or 4-halogen, 2,4-halogen, 3,4-halogen, 4-$C_{1-4}$alkyl or 4-$C_{1-4}$alkoxy; and
(iii) when n is 2, X is other than hydrogen;
which comprises reacting a compound of formula (II):

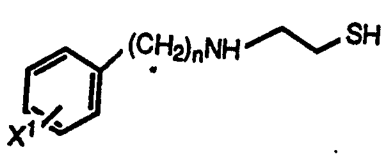

(II)

in which $X^1$ is as described as described for formula (I) but not hydroxy with a xanthate of formula (III):

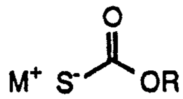

(III)

in which $M^+$ is an alkali metal ion and R is $C_{1-4}$alkyl and when compounds wherein is X is OH are desired, deprotecting the compounds wherein $X^1$ is $C_{1-4}$alkoxy.

2. A process according to claim 1 for preparing a compound in which n is 0-2.

3. A process according to claim 1 or 2 for preparing a compound in which n is 1.

4. A process according to any one of claims 1, 2, or 3 for preparing a compound in which X is one or two substitutents.

5. A process according to any one of claims 1-4 for preparing a compound in which X is one or two halogen substituents.

6. A process according to claim 1 for preparing a compound which is 3-(3-fluorophenylmethyl)-2-thiazolidinethione or 3-(3,5-difluorophenylmethyl)-2-thiazolidinethione; or a hydrate thereof.

7. A process for preparing 3-(3-fluorophenylmethyl)-2-thiazolidinethione or a hydrate thereof which comprises reacting N-(3-fluorobenzyl)-2-mercaptoethylamine with potassium ethyl xanthate.

8. A process for preparing 3-(3,5-difluorophenylmethyl)-2-thiazolidinethione or a hydrate thereof which comprises reacting N-(3,5-difluorobenzyl)-2-mercaptoethylamine with potassium ethyl xanthate.

9. A process for preparing a pharmaceutical composition which comprises bringing into association a compound according to any one of claims 1 to 6 or a hydrate thereof and a pharmaceutically acceptable carrier.

10. The use of a compound of formula (I) or a hydrate thereof as defined in claim 1 in the manufacture of a medicament for inhibiting dopamine-$\beta$-hydroxylase.